# EUROPÄISCHE PATENTSCHRIFT

(11) **EP 2 821 771 B1**
(45) Veröffentlichungstag und Bekanntmachung des Hinweises auf die Patenterteilung: **03.04.2019**
(21) Anmeldenummer: 14174592.7
(22) Anmeldetag: 26.06.2014
(51) Int. Cl.: G01N 3/08, G01N 3/32, G01N 33/00

(54) **Prüfmethode**
Testing method
Méthode de vérification

(30) Priorität: 02.07.2013 DE 102013212884
(43) Veröffentlichungstag der Anmeldung: 07.01.2015
(73) Patentinhaber: Wobben Properties GmbH, 26607 Aurich (DE)
(72) Erfinder: Hesse, Ingo, 26632 Ihlow (DE)
(74) Vertreter: Eisenführ Speiser

(56) Entgegenhaltungen:
- EP-A2- 0 037 987
- DE-A1-102010 002 131
- DE-A1-102010 026 620
- KR-A- 20110 001 645
- PALANIVELU S ET AL: "Comparison of the crushing performance of hollow and foam-filled small-scale composite tubes with different geometrical shapes for use in sacrificial cladding structures", COMPOSITES PART B, Bd. 41, Nr. 6, 1. September 2010 (2010-09-01), Seiten 434-445, XP027150816, ISSN: 1359-8368
- GUDEN M ET AL: "Effect of aluminum closed-cell foam filling on the quasi-static axial crush performance of glass fiber reinforced polyester composite and aluminum/composite hybrid tubes", COMPOSITE STRUCTURES, Bd. 81, Nr. 4, 25. Juni 2007 (2007-06-25), Seiten 480-490, XP022127664, ISSN: 0263-8223, DOI: 10.1016/J.COMPSTRUCT.2006.09.005

## Beschreibung

Die Erfindung betrifft eine Prüfmethode zur Ermittlung einer spezifischen Werkstoffeigenschaft eines Probekörpers, unter Verwendung des Probekörpers einer länglichen Probe eines faserverstärkten Kunststoffverbundes eines Rotorblatts einer Windenergieanlage, gemäß dem Oberbegriff des Anspruchs 1.

In technischen Anwendungen kommen verschiedene faserverstärkte Kunststoffverbunde zum Einsatz. Dabei bestehen die faserverstärkten Kunststoffverbunde aus Fasern und einer entsprechenden Matrix, die für die notwendige Verbundhaftung sorgt. Das Eigenschaftsprofil dieser faserverstärkten Kunststoffverbunde wird, neben der Auswahl von Fasern und Matrixwerkstoff, durch die Orientierung der Fasern im textilen Flächengebilde bestimmt. Die im Verbund auftretenden Materialien weisen meist funktionale Eigenschaften auf, die zweckgebunden hinsichtlich Ihres Einsatzgebietes sind. Für die Eigenschaften des erhaltenen Werkstoffes bzw. Verbundbauteils sind stoffliche, unter Umständen auch geometrische Eigenschaften der einzelnen Komponenten von Bedeutung. Verbundbauteile weisen meist Eigenschaften auf, die unter Lasteinwirkung ein optimiertes Verhalten des Formteiles darstellen. Die Eigenschaften können hinsichtlich angelegter mechanischer Belastungen einer gewissen Festigkeit bzw. einer gewissen Steifigkeit oder einer gewissen Dehnbarkeit zugeordnet sein.

Voraussetzung für ein faserverstärktes Kunststoffverbundbauteil ist, dass unter Lasteinwirkung das Verhalten des Verbundes eine Optimierung gegenüber den Einzelkomponenten darstellt. Die Entwicklung geht dahin, dass die geforderten Eigenschaften in Kombination zur Lebensdauer optimiert werden, um einer langjährigen, zyklischen Belastung standzuhalten.

Ein Beispiel hierfür ist ein entsprechendes faserverstärktes Bauteil eines Rotorblattes einer Windenergieanlage. Rotorblätter sind in der Regel aus entsprechenden Fasern aufgebaut, d. h., hauptsächlich Glas- und/oder Kohlefasern, in einem harzartigen Lami-Matrix-Material. Diese Fasern sind in der Regel orientiert in oder entlang der Längsachse des Rotorblattes, wobei das genaue Ausrichten der Fasern meist sehr schwierig zu kontrollieren ist in Abhängigkeit vom Herstellungsprozess. Bei Rotorblättern von Windenergieanlagen werden hauptsächlich große Lasteinwirkungen ausgeübt. Dabei sollten die Rotorblätter sowohl der statischen als auch der sehr häufigen dynamischen Belastung standhalten.

Die Werkstoffprüfung bildet in der Regel einzelne Beanspruchungsszenarien an genormten Probekörpern ab. Aufgrund der starken Richtungsabhängigkeit der Eigenschaften werden die verschiedenen Beanspruchungsarten mit unterschiedlichen Proben bzw. Probekörpern längs und quer zur Hauptfaserrichtung durchgeführt. Neben der internationalen Normung sind Prüfungen in verschiedenen nationalen oder regionalen Normen, wie auch in firmeneigenen Regelwerken beschrieben. Dadurch ergibt sich ein weites Feld, das rund 20 generische Prüfmethoden beschreibt.

Die Prüfung an Bauteilen, Strukturausschnitten und kompletten Strukturen ist in der Regel nahe an den Beanspruchungen bzw. an den Belastungen ausgerichtet, die im späteren Betrieb auftreten. Festigkeiten, Energieaufnahme, Materialermüdung und Lebensdauerabschätzung stehen im Vordergrund. Aufgrund der Richtungs- und Scherempfindlichkeit der Faserverbunde sollten Prüfkräfte möglichst in der vorgesehenen Richtung angebracht werden. Der Axialfehler wird als Schiefzug bezeichnet und unterliegt engen Grenzen. Spezielle Messeinrichtungen orientieren sich an der Form und der Dimension des Probekörpers und bieten zusätzliche Stütz- und Haltevorrichtungen an, um ein klassisches Versagen durch einen Schiefzug bzw. einen eulerschen Knick zu vermeiden. Die Ausrichtung erfolgt hierbei über mechanische Justageeinrichtungen. Die Krafteinleitung in den Probekörper sollte großflächig erfolgen, was über Krafteinleitungselemente erreicht wird. Probenhalter, die nach dem Keil- oder Keilschraubprinzip arbeiten, sind hierfür üblich. Die exakte Ausrichtung kann im einfachen Fall mit Hilfe eines Dehnungsmessstreifens kontrolliert werden. Dieser wird auf den Probekörper entsprechend appliziert und durch die gemessene Probendehnung verifiziert.

Die Messung bzw. die Ermittlung spezifischer Werkstoffeigenschaften hinsichtlich der Mechanik für Rotorblätter bzw. Rotorblätter in Windenergieanlagen setzt eine hohe Lasteinwirkung voraus. Zur Ermittlung der notwendigen Werkstoffeigenschaften sollten die entsprechenden Kräfte auf den Probekörper einwirken. Die Vorrichtungen zur Ermittlung werden meist durch zusätzliche Knickstütze ausgestattet, um ein vorrichtungs- und prüfmethodenspezifische Versagen des Bauteils zu verhindern. Das häufig auftretende Knicken bedeutet ein schlagartiges bzw. gewaltsames Versagen der Probekörper und liefert grundsätzlich nicht das aufbauspezifische Ergebnis für den jeweiligen faserverstärkten Kunststoffverbund. Die angebrachten Knickstützen bzw. Haltevorrichtungen bewirken, dass die axiale Ausrichtung gewährleistet ist und das Knicken verhindert wird, da entsprechende Druckkräfte gegen das zum knicken führende Biegemoment wirken.

Das deutsche Patent- und Markenamt hat in der Prioritätsanmeldung folgenden Stand der Technik recherchiert: DE 10 2006 035 274 A1, DE 10 2010 002 131 A1 und DE 20 2012 008 324 U1.

In dem Artikel von PALANIVELU S ET AL: "Comparison of the crushing performance of hollow and foam-filled small-scale composite tubes with different geometrical shapes for use in sacrificial cladding structures", COMPOSITES PART B, Bd. 41, Nr. 6, 1. September 2010 (2010-09-01), Seiten 434-445, XP027150816 sind Glas/Polyester-Verbundröhren mit Polyurethan Schaum im Inneren beschrieben, welche deshalb eine höhere Knickstabilität aufweisen.

In dem Artikel von GUDEN M ET AL: "Effect of aluminum closed-cell foam filling on the quasi-static axial crush performance of glass fiber reinforced polyester composite and aluminum/composite hybrid tubes", COMPOSITE STRUCTURES, Bd. 81, Nr. 4, 25. Juni 2007 (2007-06-25), Seiten 480-490, XP022127664 ist eine Prüfmethode unter Verwendung eines Probekörpers beschrieben, wobei der Effekt einer geschlossen-zelligen Aluminiumschaum-Füllung auf die Knickstabilität einer Polyester-Verbundröhre aus E-Glass-Gewebe und eine Al/Polyester-Verbund-Hybrid-Röhre experimentell untersucht wird. Der Oberbegriff des Anspruchs 1 ist im Hinblick auf den Artikel von GUDEN M ET AL formuliert.

Die vorliegende Erfindung möchte eine Alternative zu dem bisherigen Prüfmethodenaufbau anbieten, da durch das Anlegen der Knickstützen das Ergebnis verfälscht werden kann. An dieser Stelle setzt die Erfindung an, deren Aufgabe es ist, eine Prüfmethode anzubieten, die eine vereinfachte Möglichkeit bietet, mechanische Lasten bzw. spezifische Werkstoffeigenschaften im Akzeptanzbereich, insbesondere mit einem geringen Messfehler, zu messen und auch höhere Kräfte bzw. höhere Belastungen als bisher üblich zu ermöglichen. Insbesondere soll ein Probekörper einem Bauteil die nötige Stabilität geben, um tatsächliche Lasteinwirkungen und Belastungszustände nachzubilden bzw. abzubilden. Darüber hinaus soll der Probekörper eine vergleichsweise vereinfachte Prüfmethode ermöglichen, die ohne das typische versuchsbedingte Versagen die statischen und dynamischen Belastungen eines Rotorblatts einer Windenergieanlage widerspiegelt.

Die Erfindung geht von einem Probekörper aus zum Ermitteln einer spezifischen Werkstoffeigenschaft eines faserverstärkten Kunststoffverbundes bei angreifender mechanischer Belastung.

Erfindungsgemäß ist vorgesehen, dass ein innerer Kern in einen Verbund mit dem faserverstärkten Kunststoff eingebracht ist, wobei der innere Kern in einer Querachse zur mechanischen Last derart erweitert ist, dass der Verbund mit innerem Kern eine höhere Knickstabilität aufweist als ein Vergleichskörper wie der Verbund ohne inneren Kern, und dass der innere Kern derart ausgebildet ist, dass ein Einfluss auf die zu ermittelnde spezifische Werkstoffgröße des faserverstärkten Kunststoffverbundes in einem Akzeptanzbereich liegt. Ein Vergleichskörper wie der Verbund ohne inneren Kern hat insbesondere eine gleiche Fasermenge, insbesondere mit der Orientierung der Fasermenge des Verbundes und/oder einen gleichen Volumenanteil eines faserverstärkten Kunststoffverbunds.

Das Konzept der Erfindung führt auf eine Prüfmethode des Anspruchs 1.

Die Erfindung geht von der Überlegung aus, dass sich ein Probekörper, wie er derzeit im Stand der Technik verwendet wird, grundsätzlich bewährt hat, jedoch systembedingte Schwächen aufweist; dies schließt auch die Prüfmethode ein. Die Erfindung hat erkannt, dass sich der Probekörper in verbesserter Weise kompakter und dennoch geometrisch spezifisch eingestellt ist und sich gegen versuchsverfälschende Einflüsse stabilisieren kann. Erfindungsgemäß ist vorgesehen, dass der innere Kern in einer Querachse zur mechanischen Last derart erweitert ist, dass der Verbund mit innerem Kern eine höhere Knickstabilität aufweist als ein Verbund ohne inneren Kern; insbesondere hat der Vergleichskörper wie der Verbund ohne inneren Kern eine gleiche Fasermenge und/oder Volumenanteil des faserverstärkten Kunststoffverbundes wie der Verbund mit innerem Kern. Hierbei erweist sich der erfindungsgemäße Querschnitt als besonders vorteilhaft, da durch den inneren Kern dieser erweitert wird, und eine höhere Knickstabilität erreicht ist. Der größere Querschnitt verhindert bei großen Biegemomenten oder hohen Lasteinwirkungen das mechanische Versagen durch ein schlagartiges Biegen zum Ausweichen der einzubringenden Lasten. Eine Knicksicherheit, musste im Stand der Technik durch das Anbringen äußerer Führungen gelöst werden. Vorliegend wird eine Knicksicherheit durch die eigenen geometrischen Parameter des belasteten Bauteils gelöst. Es wird der Knickkraft ein axiales Flächenträgheitsmoment des Querschnitts zugrundegelegt, um eine höhere Knickbeständigkeit zu erreichen. Es ist abhängig von der Art der Beanspruchung und der Länge des jeweiligen Probekörpers sowie der Querschnittsform des Probekörpers. Erfindungsgemäß ist zusätzlich vorgesehen, dass dieser innere Kern, der den Querschnitt des Probekörpers erweitert, derart ausgebildet ist, dass ein Einfluss auf die zu ermittelnde spezifische Werkstoffeigenschaft des faserverstärkten Kunststoffverbundes in einem Akzeptanzbereich, nämlich Messfehlerbereich, liegt. Dieses erfinderische Merkmal greift den Gedanken auf, dass ein zusätzlicher Kern das gesamte Verbundbauteil hinsichtlich der zu messenden Werkstoffeigenschaft verändern kann. Hierbei wird berücksichtigt, dass es Werkstoffe gibt, die einen geringen oder einen nicht zu messenden Einfluss auf einen Verbund haben. Dieses wird für die vorliegende Erfindung genutzt, indem ein innerer Kern eingebracht wird, der einen so geringen Einfluss auf die zu ermittelnde spezifische Werkstoffeigenschaft hat, dass die ihm zuordenbare Werkstoffeigenschaft unterhalb des Fehlerbereiches liegt.

Durch die vorliegende Erfindung können große Lasten angebracht werden, die die tatsächliche Belastung z. B. einer Windenergieanlage darstellt, ohne prüftechnisch äußere Mittel anzuwenden, um ein Ergebnis in entsprechender Höhe zu erzielen. Die Erfindung gibt eine Möglichkeit, einen Verbund herzustellen, der die Prüfmethodenfehler kompensiert durch einen höheren Querschnitt, der das Knicken unter Belastungen verhindert. Es werden Materialien eingesetzt, die die zu messenden Werkstoffeigenschaften nicht beeinflussen, bzw. innerhalb des Akzeptanz-, insbesondere Messfehler-Bereiches liegen. Diese Methode ermöglicht eine Messung, die zum einen eine geringere Fehlerspannweite und geringe Ausfälle aufweist und zum anderen das Messen von faserverstärkten Kunststoffverbunden deutlich vereinfacht. Durch den inneren Kern als innere Stabilisierung wird lediglich eine höhere Knickbeständigkeit erreicht, bedeutet aber nicht wie im bisherigen Stand der Technik eine Ummantelung, bzw. Stabilisierung, die die Messung von Werkstoffverbundeigenschaft biaxial stärker beeinflussen kann. Zusätzlich können Risse bzw. Rissfortpflanzungen bei dynamischer Belastung und eintretender Materialermüdung leichter verfolgt werden und die Rissfortpflanzung wird nicht durch außenliegende Stützvorrichtungen und dessen Kraftableitung verlangsamt.

Als besonders vorteilhaft hat sich erwiesen, dass der innere Kern das empfindliche Biegeverhalten von faserverstärkten Kunststoffverbunden einschränkt und die gemessenen Zug- und Druckfestigkeiten den axialen Ausrichtungen des Faserverbundes entsprechen und den Prüfversuch entsprechend optimieren können. Die Kraftanleitung in dem Probekörper kann nun durch die axiale Ausrichtung erfolgen, ohne die zuvor geforderte hohe axiale Ausrichtung zu benötigen. Der Probekörper ermöglicht hiermit einen anwendbaren Prüfversuch, der das Versagen des Bauteils widerspiegeln kann, ohne die zu erwartenden Prüfmethodenfehler wie ein Knicken aufzuweisen.

Weitere vorteilhafte Weiterbildungen der Erfindung sind den Unteransprüchen zu entnehmen und geben im Einzelnen vorteilhafte Möglichkeiten an, das erläuterte Konzept im Rahmen der Aufgabenstellung sowie hinsichtlich weiterer Vorteile zu realisieren.

In einer bevorzugten Weiterbildung ist ein innerer Kern des Probekörpers vorgesehen, der eine innere Lage eines Schichtsystems bildet. Damit bietet diese Weiterbildung ein Einbringen des inneren Kerns in ein Fasermaterial, welches matten- oder geflechtartig ausgebildet ist, so dass der Kern in diese mattenartige Ausbildung eingefügt werden kann, ohne die geometrischen Abmaße und die fasergerichteten spezifischen Eigenschaften maßgeblich zu verändern. Diese Weiterbildung ermöglicht ein Messen mit dem eindeutigen Beobachten von dem Versagen der jeweiligen faserverstärkten Kunststoffverbunde entlang der Fasern, da dieses vorteilhafte Schichtsystem die Rissermüdung innerhalb der faserverstärkten Kunststoffverbunde im Querschnitt deutlich darstellen kann. Darüber hinaus ist diese Weiterbildung das pragmatische und vereinfachte Verfahren zur Herstellung eines Prüfkörpers. Die mattenartig gestalteten Faserverbundwerkstoffe können hierbei die geometrischen Abmaße beibehalten und werden nur zu einer Querachse hin verdickt, ohne ihre eigenen geometrischen Verläufe zu verändern. Damit kann bei dieser Weiterbildung besonders vorteilhaft beobachtet werden, ob sich in den verschiedenen Achsen die Materialeigenschaften verändern und Spannungszuwächse zu beobachten sind.

In einer bevorzugten Weiterbildung ist ein Probekörper vorgesehen, wobei der innere Kern einen geschäumten Kunststoff aufweist. Diese Weiterbildung ermöglicht das Einbringen von einem inneren Kern, der aufgrund seiner Materialbeschaffenheit und seines Herstellungsprozesses, das Schäumen, und aufgrund seiner Materialeigenschaft derartig gestaltet ist, dass der Einfluss in dem Akzeptanz-, insbesondere Messfehler-Bereich der Prüfmethode liegen wird. Um die Knicksteifigkeit und/oder die Belastungen zu erhöhen kann insbesondere dieser geschäumte Kunststoff ein Füllmaterial aufweisen, welches den inneren Kern zusätzlich stützt, nicht aber die zu messende Werkstoffeigenschaft des faserverstärkten Kunststoffverbundes verändert. Insbesondere gilt dies für einen Probekörper mit einem inneren Kern, wobei der innere Kern einen geschäumten Kunststoff mit Füllmaterial aufweist. Diese Knickbeständigkeit der zusätzlichen Füllstoffe wird ausschließlich dem inneren Kern zugute gereicht werden. Das zusätzliche Füllmaterial ermöglicht zudem eine gleiche Knickbeständigkeit bei geringerem Querschnitt gegenüber einem geschäumten Kunststoff. Es ist vorteilhaft in Abhängigkeit vom Prozess der jeweilige Werkstoff auszuwählen, welcher für den inneren Kern verwendet wird. Es ist immer zu berücksichtigen, dass die Werkstoffeigenschaften des inneren Kerns so klein sein sollten, dass sie im Akzeptanz-, insbesondere Messfehler-Bereich der Prüfmethode liegen sollten. Ansonsten würde sich das Problem ergeben, dass eine integrale Aufsummierung der Werkstoffeigenschaften bezogen auf den Prüfkörper nicht die tatsächlichen spezifischen Werkstoffeigenschaften eines faserverstärkten Kunststoffverbundes darstellen würde.

Erfindungsgemäß ist ein Probekörper vorgesehen, wobei der Probekörper mit einer rechteckigen, länglichen Probengeometrie umschreibbar ist, die das äußere Abmaß von mindestens 0,5 cm Dicke, mindestens 2 cm Breite und mindestens 20 cm Länge aufweist. Dies ist den mechanischen Lastversuchen bzw. mechanischen Prüfmethoden geschuldet, die meist längliche Proben verwenden. Hierbei sind insbesondere Zug- und Druckprüfmethoden herangezogen worden, um die Probengeometrie zu beschreiben. Die jeweilige Prüfmethode und deren Prüfapparatur bedingen die Probengeometrie vor allem in ihrer Breite und Dicke. In Abhängigkeit von der Länge kann entweder das Biegemoment oder die Einschnürungszone entstehen. Um realistische Kräfte einbringen zu können, soll eine gewisse Mindestdicke und -breite gewährleistet sein, die ermöglicht, realistische Lasten gemäß des Einsatzgebietes darzustellen. Für die faserverstärkten Kunststoffverbunde ist hierbei die Probengeometrie und der Probenaufbau wichtig, um die realen Belastungszonen der jeweiligen Anwendungsgebiete widerzuspiegeln. Die Breite ist zuständig für die Lastverteilung der Fixiereinrichtung und ist variabel in Abhängigkeit von der Prüfmethode und deren angelegten Kräften und jeweiligen Versuchsbedingungen.

Erfindungsgemäß ist ein Probekörper vorgesehen, wobei die Probengeometrie in einer Fläche zulaufende Enden aufweist, die entsprechend einer Fixiereinrichtung angepasst sind. Diese Weiterbildung berücksichtigt zum einen die jeweilige Prüfmethode und deren Fixiereinrichtung, zum anderen die funktionale Erweiterung des Querschnittes durch einen inneren Kern hinsichtlich eines bestimmten Biegemomentes; es können im Prinzip auch andere Enden vorgesehen sein, z.B. auch sich erweiternde, oder solche anderen Querschnitts wie runde, ovale, oder eckige Querschnitte.. Der Querschnitt sollte in dem Bereich, wo die höchsten Lasteinwirkungen auftreten, erweitert werden. Es wird der innere Kern, wie von der Weiterbildung erkannt, zumeist nicht an den Enden benötigt, sondern an den höchsten Lastwirkungszonen. Diese treten bevorzugt in der Mitte der Probe auf, wenn symmetrisch an beiden Enden die Lasteinwirkung erfolgt. Damit können aufgrund dieser Weiterbildung bisherige Versuchsaufbauten genutzt werden und diese auch ohne zusätzliche äußere Befestigungen oder Halterungsringe. Der innere Kern, dessen Einbringung von Materialeigenschaften im Akzeptanz-, insbesondere Messfehler-Bereich liegen, bringt vorteilhaft keine zusätzliche Änderung der Prüfmethode bzw. der Prüfvorrichtung mit. Diese Weiterbildung führt so zu einer Vereinfachung der bisher angewendeten Prüfmethoden.

Erfindungsgemäß ist vorgesehen, dass ein faserverstärkter Kunststoffverbund mindestens einen Dehnmessstreifen aufweist. Dies spiegelt bisherige Prüfmethoden wider. Allerdings liegt hier nicht ein Beobachten der Axialen Ausrichtung vor, um die Prüfmethode abzusichern. Sondern stattdessen wird hier anhand von Dehnmessstreifen Veränderungen der Fasern beobachtet, welche zum das Verteilen der anliegenden Kraft darstellen und zum anderen aber die Materialermüdung beobachten lassen. Diese Dehnmessstreifen können nun an dem Probekörper für dynamische und statische Lasteinwirkungen aufgebracht werden den jeweiligen Versuchsaufbau hinsichtlich seiner axialen Durchführung zu beobachten.

In einer bevorzugten Weiterbildung ist ein Probekörper vorgesehen, wobei die mechanischen Belastungen einer Zugbelastung und/oder Druckbelastung entsprechen. Diese Weiterbildung greift die Prüfmethoden für faserverstärkte Kunststoffe hinsichtlich ihres Einsatzgebietes auf und bringt als mechanische Last Zug- oder Druckbelastung auf, die diesen Probekörper durch seinen faserverstärkten Kunststoffverbund mit zusätzlich stabilisierendem inneren Kern entsprechend belasten. Dabei ist der Probekörper mit einem gleichbleibenden charakteristischen Geometrieverhältnis notwendig, um bei gleichen Versuchsbedingungen und unterschiedlichen Materialien die jeweilige Belastungsgrenze darstellen zu können und bei gleichen faserverstärkten Kunststoffverbunden aber unterschiedlichen Querschnitten bzw. unterschiedlichen Faserorientierungen Unterschiede bei gleicher Belastung darstellen zu können. Der Probekörper ermöglicht zudem bei Zug- oder Druckbelastungen eine erhöhte Stabilität gegenüber dem bisherigen Verjüngen des Querschnittes bzw. des Knickens. Der Füllstoff wird hierbei nicht das Ergebnis verfälschen, da er im jeweiligen Akzeptanz-, insbesondere Messfehler-Bereich der jeweiligen Versuchsbedingung liegt.

In einer bevorzugten Weiterbildung ist ein Probekörper vorgesehen, wobei die mechanischen Belastungen einer dynamischen Belastung entsprechen. Diese Weiterbildung ist dem Umstand geschuldet, dass in der Realität an Bauteilen mit faserverstärkten Kunststoffverbunden meist dynamische Kräfte anliegen. Diese dynamischen Kräfte haben einen deutlichen Einfluss auf die Lebensdauer eines Bauteils und dessen Beständigkeit. Der vorliegende Probekörper ermöglicht es dynamische Belastungen aufzubringen, ohne Messfehler bzw. frühzeitige Ausfälle durch ein Knickversagen darzustellen, die die Bewertung des faserverstärkten Kunststoffverbundes negativ beeinflussen. Erfindungsgemäß ist eine Prüfmethode vorgesehen unter Verwendung des Probekörpers. Dies führt dazu, dass der Probekörper derartig eingesetzt werden kann, dass die bisher bekannten Prüfmethoden verwendet und den Anwendungsgebieten angepasst werden können. Hierbei sind sowohl höhere dynamische als auch höhere statische Belastungen möglich, da die Ausfallswahrscheinlichkeit durch den zusätzlichen inneren Kern verringert wird und das Prüfergebnis einen geringeren Messfehler-Bereich aufweist. Die Prüfmethode kann durch den jeweiligen Prüfkörper und dessen Geometrie spezifisch angepasst werden und nutzt die Vorteile des Probekörpers mit entsprechend innerem Kern. Dabei spiegeln die ermittelten spezifischen Werkstoffeigenschaften die jeweiligen faserverstärkten Kunststoffverbunde wider, ohne durch ein zusätzliches Element wie dem inneren Kern verfälscht zu werden.

Erfindungsgemäß wird der Probekörper zur Prüfung eines faserverstärkten Kunststoffverbundes eines Bauteils einer Windenergieanlage verwendet, nämlich eines Rotorblattes. Dies bezieht sich darauf, dass vor allem statische und dynamische Belastungen an Windenergieanlagen insbesondere an den Rotorblättern auftreten. Damit sollten hier besondere Prüfmethoden bzw. genaue Prüfmethoden angewandt werden, um die jeweiligen statischen und dynamischen Belastungen widerspiegeln zu können und Materialeigenschaften darstellen zu können. Der Probekörper bietet die Gelegenheit, neben der Materialauswahl auch die Prüfmethode hinsichtlich höherer Kräfte zu verändern, welches eine Weiterentwicklung von Rotorblättern ermöglichen könnte. Die angreifenden Kräfte, die derzeit die versuchstechnisch eingeschränkt sind, können nun in tatsächliche Werte überführt werden, da die Ausfallswahrscheinlichkeit, bedingt durch den zu schmalen Querschnitt, verringert wurde. Der Probekörper
liefert für eine Prüfmethode für ein jeweiliges Rotorblatt genaue und realistische Werte des jeweiligen faserverstärkten Kunststoffverbundes.

Ausführungsbeispiele der Erfindung werden nachfolgend anhand der Zeichnung im Vergleich zum Stand der Technik, welcher zum Teil ebenfalls dargestellt ist, beschrieben. Diese soll die Ausführungsbeispiele nicht notwendigerweise maßgeblich darstellen, vielmehr ist die Zeichnung, wo zur Erläuterung dienlich, in schematisierter und/oder leicht verzerrter Form ausgeführt. Im Hinblick auf Ergänzungen der aus der Zeichnung unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in der Zeichnung sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, der Zeichnung und/oder den Ansprüchen offenbarten Merkmale. Die allgemeine Idee der Erfindung ist nicht beschränkt auf die exakte Form oder das Detail der im Folgenden gezeigten und beschriebenen bevorzugten Ausführungsform oder beschränkt auf einen Gegenstand, der eingeschränkt wäre im Vergleich zu dem in den Ansprüchen genannten Gegenstand. Bei angegebenen Bemessungsbereichen sollen auch innerhalb der genannten Grenzen liegende Werte als Grenzwerte offenbart und beliebig einsetzbar und beanspruchbar sein. Weitere Vorteile, Merkmale und Einzelheiten der Erfindung ergeben sich aus der nachfolgenden Beschreibung der bevorzugten Ausführungsbeispiele sowie anhand der Zeichnungen. Im Einzelnen zeigt die Zeichnung in:
- Fig. 1: eine schematisierte Darstellung der Probenform;
- Fig. 2: eine schematisierte Darstellung des Probekörpers in einem bevorzugten Ausführungsbeispiel;
- Fig. 3: eine schematisierte Darstellung einer Einspannvorrichtung für eine Prüfmethode in einem bevorzugten Ausführungsbeispiel.

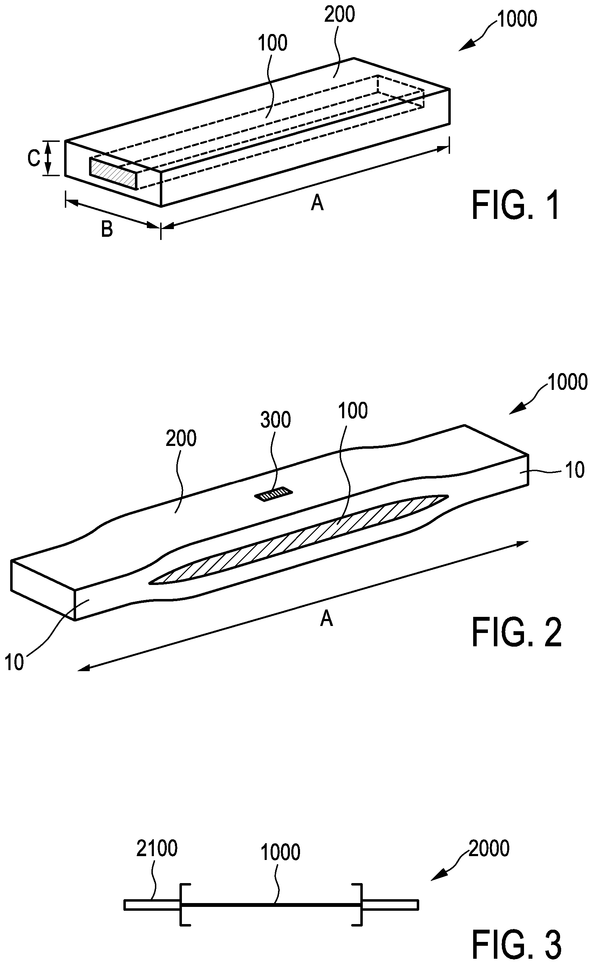

In Fig. 1 sind die äußeren geometrischen Abmaße des Probekörpers 1000 mit innerem Kern 100 und äußerem faserverstärkten Kunststoffverbund 200 anhand von Buchstaben dargestellt, wobei der charakteristische Eindruck einer rechtwinkligen länglichen Probe entsteht. Die Länge A, welche mindestens 20 cm aufweisen sollte, ist grundsätzlich vorteilhaft deutlich höher als die Breite charakterisiert durch den Buchstaben B. Der Probekörper ist in der Höhe, charakterisiert durch den Buchstaben C, mindestens 0,5 cm dick. Die hier dargestellte Probe stellt den faserverstärkten Kunststoffverbund in Form eines Probekörpers 1000 dar, der herangezogen wird, um mechanische Belastungen wie z. B. Zug oder Druck widerspiegeln zu können. Die ermittelte spezifische Werkstoffeigenschaft kann durch die charakteristische Probengeometrie dargestellt werden. Dabei ist der Einfluss der Probengeometrie auf das jeweilige Ergebnis in Abhängigkeit von den Prüfbedingungen gegeben. Der hier vorliegende Probekörper 1000 zeigt die typischerweise dargestellten faserverstärkten Kunststoffverbund 200, welche in einer axialen Ausrichtung eine gewisse Länge, hingegen in der den weiteren Achsen meist geringere Höhen aufweisen.

In Fig. 2 wird ein Probekörper 1000 schematisch dargestellt, wobei die hier charakteristische Probengeometrie dahingehend modifiziert wurde, dass der innere Kern 100 gegenüber dem faserverstärkten Kunststoffverbund in der Längsachse 200 dargestellt ist. Der faserverstärkte Kunststoffverbund liegt hier als Schichtsystem vor, welcher den inneren Kern als innere Schicht mit einschließt. Die jeweiligen Enden 10 laufen aufeinander zu und weisen für die Einspannvorrichtung entsprechend nur noch den faserverstärkten Kunststoffverbund auf. Der hier eingebrachte innere Kern 100 ist ein zusätzliches Material, welches den Querschnitt erweitert für jeweilige Belastungsmessungen, ohne das Prüfergebnis zu verfälschen. Der Querschnitt dient dazu, eine bestimmte Beständigkeit entlang, möglichst parallel, zur Faserorientierung (Achse A) zu erreichen. Die zu messende Größe, die Werkstoffeigenschaften in axialer Ausrichtung des faserverstärkten Kunststoffverbundes, wird durch diesen Kern nicht beeinflusst, sondern nur gegen das Knicken stabilisiert. Der an der Oberfläche des Probekörpers 1000 applizierte Dehnmessstreifen 300 kann die Veränderung der Längeneinheiten durch angelegte Lasten messen.

In Fig. 3 ist eine Prüfvorrichtung 2000 stilisiert in Form von einer angreifenden Fixiereinrichtung, die einaxial von beiden Seiten angreift. Der Probekörper 1000 ist hierbei an beiden Enden in die Fixiereinrichtung des Prüfstandes 2100 eingespannt und erfährt eine gerichtete Belastung, die sich im Bauteil verteilen wird, so dass der innere Kern Ausfällen wie Knicken bzw. Querschnittsverdünnung entgegenwirken kann. Die anliegenden Kräfte entsprechend der Prüfmethode können hierbei statische lasten wie Zug oder Druckspannungen sein oder dynamische Belastungen. Vorzugweise die Versuchsaufbauten für faserverstärkte Kunststoffverbunde, die in einer Windenergieanlage verwendet werden, insbesondere in einem Rotorblatt, sind mit der Prüfvorrichtung stilisiert.

### Bezugszeichenliste

- 10: Enden des Probekörpers
- 100: innerer Kern
- 200: faserverstärkter Kunststoffverbund
- 300: Dehnmessstreifen
- 1000: Probekörper
- 2000: Prüfvorrichtung
- 2100: Fixiereinrichtung

## Patentansprüche

1. Prüfmethode zur Ermittlung einer spezifischen Werkstoffeigenschaft eines Probekörpers
- unter Verwendung des Probekörpers (1000) einer länglichen Probe eines faserverstärkten Kunststoffverbundes (200) eines Rotorblattes einer Windenergieanlage, wobei der Probekörper (1000) eine Länge (A) entlang einer Längsachse und einen Querschnitt mit einer Breite (B) und einer Dicke (C) in einer Querachse hat und wobei der Probekörper (1000) den faserverstärkten Kunststoffverbund (200) und einen inneren Kern aufweist, wobei
- die spezifische Werkstoffeigenschaft des faserverstärkten Kunststoffverbundes (200) ermittelt wird, nämlich indem eine mechanische statische oder dynamische Last einer Zugbelastung und/oder Druckbelastung entlang der Längsachse des Probekörpers (1000) in einer Fixiereinrichtung (200) einer Prüfvorrichtung (2000) in einer Messung am Probekörper (1000) angreift und die spezifische Werkstoffeigenschaft ermittelt wird, wobei
- der innere Kern (100) in einen Verbund mit dem faserverstärkten Kunststoffverbund (200) eingebracht wird, **dadurch gekennzeichnet, dass**
- durch den inneren Kern in der Querachse zur mechanischen Last der Querschnitt der Probe derart erweitert wird, dass der Verbund mit dem inneren Kern eine höhere Knickstabilität aufweist als ein Verbund ohne den inneren Kern, wobei
- der Probekörper mit einer im Wesentlichen rechteckigen, länglichen Probengeometrie umschreibbar ist, und die Probengeometrie ein äußeres Abmaß von mindestens 0,5 cm Dicke (C), mindestens 2 cm Breite (B) und mindestens 20 cm Länge (A) aufweist, und
- der faserverstärkte Kunststoffverbund mindestens einen Dehnmessstreifen (300) aufweist, und
- der innere Kern (100) derart ausgebildet wird, dass ein Einfluss auf die zu ermittelnde spezifische Werkstoffeigenschaft des faserverstärkten Kunststoffverbundes derart gering ist, dass die dem Kern zuzuordnende Werkstoffeigenschaft in einem Messfehlerbereich der Messung für die spezifische Werkstoffeigenschaft liegt, wobei der Querschnitt der Probe im Bereich höchster Lasteinwirkung in der Mitte der Probe erweitert wird, und die Probengeometrie in einer Fläche zulaufende Enden (10) aufweist, die entsprechend der Fixiereinrichtung angepasst sind.

2. Prüfmethode nach Anspruch 1, wobei der innere Kern (100) des Probekörpers (1000) eine innere Lage eines Schichtsystems des faserverstärkten Kunststoffverbundes bildet.

3. Prüfmethode) nach Anspruch 1 oder 2, wobei der innere Kern (100) des Probekörpers (1000) einen geschäumten Kunststoff aufweist.

4. Prüfmethode (1000) nach Anspruch 3, wobei der innere Kern (100) des Probekörpers (1000) einen geschäumten Kunststoff mit Füllmaterial aufweist.

## Claims

1. Testing method for establishing a specific material property of a test specimen
- using the test specimen (1000) of an elongate specimen of a fibre-reinforced plastics material composite (200) of a rotor blade of a wind turbine, wherein the test specimen (1000) has a length (A) along a longitudinal axis and a cross-section having a width (B) and a thickness (C) in a transverse axis and wherein the test specimen (1000) has the fibre-reinforced plastics material composite (200) and an inner core, wherein
- the specific material property of the fibre-reinforced plastics material composite (200) is established, that is to say, by a mechanical, static or dynamic load of a tensile load and/or pressure load along the longitudinal axis of the test specimen (1000) engaging in a fixing device (200) of a testing device (2000) in a measurement on the test specimen (1000) and the specific material property being established, wherein
- the inner core (100) is introduced into a composite with the fibre-reinforced plastics material composite (200), **characterised in that**,
- as a result of the inner core in the transverse axis with respect to the mechanical load, the cross-section of the specimen is expanded in such a manner that the composite with the inner core has a higher resistance to kinking than a composite without the inner core, wherein
- the test specimen can be described with a substantially rectangular, elongate specimen geometry and the specimen geometry has an outer dimension with a thickness of at least 0.5 cm (C), a width (B) of at least 2 cm and a length (A) of at least 20 cm, and
- the fibre reinforced plastics material composite has at least one strain gauge (300), and
- the inner core (100) is constructed in such a manner that an influence on the specific material property of the fibre-reinforced plastics material composite which is intended to be established is so low that the material property which is intended to be associated with the core is located within a measurement error range of the measurement for the specific material property, wherein the cross-section of the specimen is expanded in the range of the maximum load application in the centre of the specimen, and the specimen geometry has ends (10) which taper into a face and which are adapted in accordance with the fixing device.

2. Testing method according to claim 1, wherein the inner core (100) of the test specimen (1000) forms an inner layer of a layer system of the fibre-reinforced plastics material composite.

3. Testing method according to claim 1 or claim 2, wherein the inner core (100) of the test specimen (1000) has a foamed plastics material.

4. Testing method (1000) according to claim 3, wherein the inner core (100) of the test specimen (1000) has a foamed plastics material with filler material.

## Revendications

1. Méthode d'essai servant à déterminer une propriété de matériau spécifique d'un corps d'échantillon
- en utilisant le corps d'échantillon (1000) d'un échantillon allongé d'un composite en matière plastique (200) renforcé par des fibres d'une pale de rotor d'une éolienne, dans laquelle le corps d'échantillon (1000) a une longueur (A) le long d'un axe longitudinal et une section transversale avec une largeur (B) et une épaisseur (C) dans un axe transversal et dans laquelle le corps d'échantillon (1000) présente le composite en matière plastique (200) renforcé par des fibres et une partie centrale intérieure,
dans laquelle
- la propriété de matériau spécifique du composite en matière plastique (200) renforcé par des fibres est déterminée, et ce qu'une charge mécanique statique ou dynamique d'une contrainte de traction et/ou d'une contrainte de pression s'engage au niveau du corps d'échantillon (1000) le long de l'axe longitudinal du corps d'échantillon (1000) dans un système de blocage (200) d'un dispositif d'essai (2000) lors d'une mesure et la propriété de matériau spécifique est déterminée, dans laquelle
- la partie centrale intérieure (100) est introduite dans un composite avec le composite en matière plastique (200) renforcé par des fibres, **caractérisée en ce que**
- la section transversale de l'échantillon est élargie à travers la partie centrale intérieure dans l'axe transversal par rapport à la charge mécanique de telle manière que le composite avec la partie centrale intérieure présente une stabilité au pliage plus élevée qu'un composite sans la partie centrale intérieure, dans laquelle
- le corps d'échantillon peut être décrit avec une géométrie d'échantillon allongée sensiblement rectangulaire, et la géométrie d'échantillon présente une dimension extérieure d'au moins 0,5 cm d'épaisseur (C), d'au moins 2 cm de largeur (B) et d'au moins 20 cm de longueur (A), et
- le composite en matière plastique renforcé par des fibres présente au moins une jauge de contrainte (300), et
- la partie centrale intérieure (100) est réalisée de telle manière qu'une incidence sur la propriété de matériau spécifique à déterminer du composite en matière plastique renforcé par des fibres est faible de telle manière que la propriété de matériau à associer à la partie centrale se situe dans une plage d'erreurs de mesure de la mesure pour la propriété de matériau spécifique, dans laquelle la section transversale de l'échantillon est élargie dans la plage de l'action de charge maximale au centre de l'échantillon, et la géométrie d'échantillon présente des extrémités (10) convergeant dans une face, qui sont adaptées conformément au système de blocage.

2. Méthode d'essai selon la revendication 1, dans laquelle la partie centrale intérieure (100) du corps d'échantillon (1000) forme une couche intérieure d'un système de couches du composite en matière plastique renforcé par des fibres.

3. Méthode d'essai selon la revendication 1 ou 2, dans laquelle la partie centrale intérieure (100) du corps d'échantillon (1000) présente une matière plastique expansée.

4. Méthode d'essai (1000) selon la revendication 3, dans laquelle la partie centrale intérieure (100) du corps d'échantillon (1000) présente une matière plastique expansée avec du matériau de remplissage.
